# EUROPEAN PATENT APPLICATION

(11) **EP 4 039 107 A1**
(43) Date of publication of application: **10.08.2022**
(21) Application number: 20870634.1
(22) Date of filing: 02.10.2020
(51) Int. Cl.: A23L 31/15

(54) **METHOD FOR PRODUCING YEAST EXTRACT**

(30) Priority: 03.10.2019 JP 2019182827
(71) Applicant: Amano Enzyme Inc., Nagoya-shi Aichi 460-8630 (JP); Amano Enzyme U.S.A. Co., Ltd., Elgin, Illinois 60124 (US); Amano Enzyme Europe Ltd., Oxfordshire, OX75SR (GB)
(72) Inventor: OKUDA, Keita, Elgin, Illinois 60124 (US); FUJIOKA, Hiroki, Norton, Oxfordshire, OX75SR (GB)
(74) Representative: Müller-Boré & Partner Patentanwälte PartG mbB
(86) International application number: PCT/JP2020/037506
(87) International publication number: WO 2021/066130

(57) **Abstract**

It is an object of the present invention to achieve a simplified process of producing a yeast extract, improved production efficiency, reduced production costs, and the like. A yeast cell lysate is treated with a ribonuclease and AMP-deaminase simultaneously to improve production efficiency. Use of heat-resistant AMP-deaminase allows reactions with the ribonuclease and the AMP-deaminase to proceed simultaneously in an efficient manner.

## Description

### Technical Field

The present invention relates to a yeast extract. More particularly, the present invention relates to a method for producing a yeast extract.

### Background Art

Nucleic acid-based seasonings typified by yeast extracts are used in various foods and the like for imparting or enhancing umami (delicious taste) and rich taste. Yeast extracts are roughly classified into those of high amino acid type that are rich in amino acids and those of high nucleic acid type that have a high nucleic acid content. The main taste components in the latter, i.e., nucleic acid-based yeast extracts, are 5'-guanylic acid (GMP) and 5'-inosinic acid (IMP). To augment or enhance the umami taste, for example, after a yeast is lysed, ribonucleic acid is degraded by a ribonuclease into nucleotides to produce GMP, while 5'-adenylic acid (AMP) produced by the nuclease treatment is converted by AMP-deaminase to IMP.

Various studies focusing on the taste components (GMP and IMP) have been made with a view toward improving the nucleic acid-based seasonings (see, for example, Patent Literatures 1 to 3).

### Citation List

### Patent Literature

Patent Literature 1: JP H06-113789 A
Patent Literature 2: WO 2015/141531
Patent Literature 3: WO 2003/055333

### Summary of Invention

### Technical Problem

Prior methods for producing yeast extracts, which involve AMP-deaminase treatment after nuclease treatment, require a complicated production process, and thus, there is much room for improvement in terms of production efficiency, production costs, and the like. Accordingly, it is an object of the present invention to achieve a simplified process of producing a yeast extract, improved production efficiency, reduced production costs, and the like.

### Solution to Problem

As a result of extensive study to achieve the foregoing object, the inventors of the present invention decided to utilize AMP-deaminase to carry out simultaneous reactions with a ribonuclease and the AMP-deaminase to achieve a simplified production process. After verifying the effectiveness of this strategy, the inventors have found that simultaneous reactions of the two enzymes result in sufficient reactions of the two enzymes, and allow a yeast extract rich in the umami components to be efficiently produced. Surprisingly, the inventors have also observed an unexpected effect in that the yield of the yeast extract is increased, and the content of the umami components (GMP and IMP) in the yeast extract (in other words, the yield of the umami components) increases. That is, it has been revealed that the simultaneous reactions of the two enzymes not only enable simplification of the production process, but are also effective in improving the yield and moreover the quality of the yeast extract.

The following inventions are based on the foregoing outcomes and analysis.
[1] A method for producing a yeast extract, comprising the step of treating a yeast cell lysate with a ribonuclease and AMP-deaminase simultaneously.
[2] The method according to [1], wherein the ribonuclease is a ribonuclease derived from *Penicillium citrinum.*
[3] The method according to [1] or [2], wherein the AMP-deaminase is AMP-deaminase derived from an actinomycete.
[4] The method according to [3], wherein the actinomycete is an actinomycete of the genus *Streptomyces.*
[5] The method according to [4], wherein the actinomycete of the genus *Streptomyces* is *Streptomyces murinus, Streptomyces celluloflavus,* or *Streptomyces griseus.*
[6] The method according to any one of [1] to [5], wherein treatment conditions are a temperature of 50 to 70°C and a pH of 4.5 to 8.0.
[7] The method according to any one of [1] to [6], wherein the yeast cell lysate is a lysate of a yeast of the genus *Saccharomyces,* a yeast of the genus *Candida,* a yeast of the genus *Kluyveromyces,* a yeast of the genus *Pichia,* a yeast of the genus *Debaryomyces,* or a yeast of the genus *Zygosaccharomyces.*
[8] A yeast extract obtained by the method according to any one of [1] to [7].
[9] A food or drink comprising a yeast extract obtained by the method according to any one of [1] to [7].

### Description of Embodiments

The present invention relates to a method for producing a yeast extract. The yeast extract obtained by the method of the present invention is typically used in a nucleic acid-based seasoning. "Nucleic acid-based seasoning" refers to a composition that contains a nucleic acid and/or a nucleotide as a taste component, and is used for seasoning. As used herein, "seasoning" includes adjusting, changing, and enhancing the taste. "Taste component" refers to a substance that causes the taste to be perceived.

In the method of the present invention, the step of treating a yeast cell lysate with a ribonuclease and AMP-deaminase simultaneously is carried out. The yeast in the yeast cell lysate, i.e., the yeast used as a raw material of the yeast cell lysate, is not limited unless it is unsuitable for use in foods. The yeast may be any of those used in the food industry, for example, yeasts of the genus *Saccharomyces,* such as *Saccharomyces cerevisiae* and *Saccharomyces pastorianus;* yeasts of the genus *Candida,* such as *Candida utilis;* yeasts of the genus *Kluyveromyces,* such as *Kluyveromyces lactis* and *Kluyveromyces marxianus;* yeasts of the genus *Pichia,* such as *Pichia pastoris;* yeasts of the genus *Debaryomyces,* such as *Debaryomyces hansenii*; and yeasts of the genus *Zygosaccharomyces,* such as *Zygosaccharomyces mellis.* Yeast collected after brewing of beer, sake, or the like may also be used. Yeast obtained by subjecting the collected yeast to a drying treatment (dried yeast) may also be used.

"Yeast cell lysate" refers to a treated product of yeast that has been subjected to a treatment that involves disruption of the cell wall. The yeast cell lysate can be prepared by subjecting the yeast to a lysis treatment. Examples of methods of subjecting the yeast to a lysis treatment include, but are not limited to: an enzymatic degradation method; an autolysis method (for example, the yeast slurry is adjusted to a pH of about 4 to 7, and then heated to 40 to 60°C and incubated for 5 to 24 hours, without inactivating the yeast); an alkali extraction method; a hot water extraction method (for example, the yeast slurry is adjusted to a pH of about 4 to 7, and then heated to 60 to 80°C and incubated for 5 to 24 hours); an acid degradation method; an ultrasonic disruption method; disruption with a homogenizer; and a freeze-thaw method. In the production of the yeast cell lysate, a single lysis treatment only may be carried out, or a combination of two or more lysis treatments may be carried out. The yeast may be cultured using a conventional method.

In one preferred embodiment, the cultured yeast is heat-treated and then treated using an enzymatic degradation method to obtain an enzyme lysate. Conditions for the heat treatment may be, for example, 80 to 90°C for 5 to 30 minutes. The enzyme used for the enzymatic degradation method may be any lytic enzyme that can lyse the cell wall of the yeast, and may be any of various lytic enzymes (for example, plant-derived enzymes such as papain and bromelain, and microorganism-derived enzymes such as bacterial proteases (for example, alcalase) and β-glucanase). Specific examples of lytic enzymes include YL-T "Amano" L (Amano Enzyme Inc.). The reaction conditions may be set to be optimum or suitable for the lytic enzyme to be used, and specific examples include a temperature of 50 to 60°C and a pH of 5.0 to 8.0. The reaction time is also not limited, and may be set to, for example, 3 to 5 hours.

The yeast cell lysate is subjected to simultaneous treatment with a ribonuclease and AMP-deaminase. That is, in the present invention, the ribonuclease and the AMP-deaminase together act on the enzyme lysate, which causes the production of nucleotides such as GMP and AMP by the degradation of the ribonucleic acid in the yeast cell lysate (the action of the ribonuclease) and the conversion of AMP to IMP (the action of the AMP-deaminase) to proceed simultaneously. Before this simultaneous treatment, some or all of insoluble components (such as the yeast cell wall) may be removed from the yeast cell lysate. Insoluble components can be removed by using, for example, a solid-liquid separation method, centrifugation, sedimentation, filtration, decantation, or compression.

To carry out simultaneous treatment with the ribonuclease and the AMP-deaminase, typically, the ribonuclease and the AMP-deaminase are added to the yeast cell lysate in the presence of water, and reacted under predetermined conditions. The ribonucleic acid in the yeast cell lysate is degraded by the ribonuclease to produce nucleotides such as GMP that is a taste component. The ribonuclease to be used is not limited, and may be a microorganism-derived ribonuclease, a plant-derived ribonuclease, or the like. Preferably, the ribonuclease is a ribonuclease derived from *Penicillium citrinum.* Specific examples of ribonucleases derived from *Penicillium citrinum* include Enzyme RP-1G (Amano Enzyme Inc.), Nuclease "Amano" G (Amano Enzyme Inc.), and Nuclease E "Amano" 7 (Amano Enzyme Inc.). The amount (activity) of the ribonuclease to be added is not limited as long as the amount of activity can sufficiently degrade the ribonucleic acid in the yeast cell lysate, for example, 0.1 to 1000 U, preferably 1 to 100 U, more preferably 2 to 50 U, and still more preferably 5 to 30 U, per gram dry weight of the yeast cell lysate to be treated. As used herein, the ribonuclease activity is defined in terms of 1 U taken as the amount of the enzyme that releases 1 µmol of phosphate per minute when it acts with AMP as the substrate at the optimum temperature and optimum pH for the enzyme.

The AMP-deaminase is used to convert AMP produced by the action of the ribonuclease to IMP. In other words, tasty IMP is produced by the action of the AMP-deaminase.

The AMP-deaminase is an enzyme that hydrolyzes AMP to produce IMP and ammonia. The AMP-deaminase to be used is not limited as long as it is suitable for simultaneous treatment with the ribonuclease, and may be, for example, AMP-deaminase produced by microorganisms of the genus *Streptomyces* (for example, *Streptomyces murinus*), the genus *Aspergillus* (for example, *Aspergillus niger*), and the like. Preferred AMP-deaminase may be AMP-deaminase with a reaction temperature of about 40 to 70°C and high thermostability. Specific examples of such AMP-deaminase with high thermostability include AMP-deaminase produced by actinomycetes of the genus *Streptomyces* (specific examples include *Streptomyces murinus, Streptomyces celluloflavus,* and *Streptomyces griseus*) (see, for example, WO 2005/105991). AMP-deaminase derived from *Streptomyces murinus* (offered by Amano Enzyme Inc. under the name of Deamizyme T) exhibits high stability, i.e., is stable at a temperature of 65°C or less (see WO 2005/105991). The amount (activity) of the AMP-deaminase to be added is not limited as long as the amount of activity can sufficiently degrade the produced AMP into IMP, for example, 100 to 200,000 U, preferably 1,000 to 100,000 U, more preferably 5,000 to 50,000 U, and still more preferably 10,000 to 30,000 U, per gram dry weight of the yeast cell lysate to be treated. The AMP-deaminase activity is the enzyme activity measured based on a decrease in optical concentration at 265 nm (OD 265) during the reaction as an index. Specifically, the method of measuring the AMP-deaminase activity is as follows: First, 1 ml of a sample solution containing AMP-deaminase is added to 3 ml of a 1:2 solution mixture of 0.017 M 5'AMP-2Na and 1/15 M phosphate buffer (pH 5.6) to obtain a reaction solution, which is reacted at 37°C for 15 minutes. After 15 minutes, a 2% perchloric acid solution is added to stop the reaction, and then 100 µl of the reaction solution is taken. Water is added to the taken 100 µl of the reaction solution to make a volume of 5 ml, and OD 265 is measured. The value similarly measured at a reaction time of 0 minute is used as a blank. Under the above-described conditions, 1 U is defined as the amount of the enzyme that reduces the absorbance difference by 0.001 in 60 minutes of the reaction.

The reaction conditions during simultaneous treatment with the ribonuclease and the AMP-deaminase are not limited as long as they allow both enzymes to act. The reaction conditions are, for example, a temperature of 50 to 70°C and a pH of 4.5 to 8.0, and preferably a temperature of 55 to 65°C and a pH of 5.0 to 6.5. The reaction time is, for example, 1 to 24 hours, and preferably 2 to 12 hours.

The product obtained by simultaneous treatment with the ribonuclease and the AMP-deaminase may be optionally subjected to a heat treatment to inactivate the ribonuclease and the AMP-deaminase. The conditions for the heat treatment to inactivate the ribonuclease and the AMP-deaminase are, for example, 10 to 60 minutes at 90 to 100°C.

The product obtained by simultaneous treatment with the ribonuclease and the AMP-deaminase, either as is or after the heat treatment, can be applied to various uses as a nucleic acid-based seasoning (for example, a yeast extract) or the like; however, it may also be subjected to an additional process such as a purification process (for example, filtration or centrifugation), a concentration process (for example, evaporation concentration, freeze concentration, or membrane concentration), or a drying process (for example, freeze drying or spray drying).

In accordance with the method of the present invention, a liquid or solid (typically as a powder, granules, or the like) yeast extract can be obtained. The yeast extract obtained by the method of the present invention can be used to enhance or adjust the tastes of various foods and drinks. Examples of foods and drinks to which the yeast extract can be applied include processed marine products (e.g., tube-shaped fish paste cake, fish paste cake, fish minced and steamed, shredded and dried squid, dried fish, salted fish guts, a fish sausage, foods boiled in soy sauce, canned food, etc.), processed meat products (e.g., ham, bacon, sausage, jerky, corned beef, restructured meat, etc.), processed vegetable products (e.g., canned or bottled vegetable, processed tomato, processed mushroom, pickled vegetables, dried vegetables, vegetable boiled down in soy sauce, etc.), noodles and breads (e.g., various noodles, bread, a sweet roll, etc.), processed grains (e.g., cereal, oatmeal, muesli, processed rice, wheat-gluten bread, barley tea, etc.), dairy products (e.g., milk, processed milk, milk bevarage, concentrated milk, powdered milk, condensed milk, fermented milk, lactic acid bacteria beverage, butter, cheese, ice cream, etc.), processed fruit products (e.g., canned or bottled fruits, jam, marmalade, dried fruits, etc.), confectioneries and desserts (e.g., biscuits, baked pastries, rice confectioneries, fried snacks, Japanese-style uncooked confectioneries, unbaked cakes, semi-perishable confectioneries, Japanese dry confectioneries, candy, chocolate, chewing gum, snack food, frozen desert, etc.); drinks (e.g., a soft drink, a carbonated drink, a fruit juice, coffee-based drinks, a vegetable juice drink, tea-based drinks, a nonalcoholic drink, alcoholic drinks, etc.), seasonings (e.g., sauce, broth, dressing, sauce, etc.), soups, roux (e.g., curry roux, stew roux, etc.); nutrition-supplement foods and drinks (e.g., protein powder, protein drinks, supplement, nutritional drink, etc.), pet food, and nutritional supplements for pets.

When the yeast extract obtained by the method of the present invention is added to a food or drink, the amount of the yeast extract to be added may be set appropriately according to the type of food or drink, the desired taste, and the like; for example, it is 0.001 to 5% by mass, and preferably 0.01 to 1% by mass, per total amount of the food or drink.

### Examples

With a view toward simplifying the process of producing a yeast extract, the following analysis was conducted.

### <Method>

### 1. Conventional Method (Separate Treatments: Nuclease Treatment Followed by Deaminase Treatment)

### (1) Lysis Treatment

13.5 g of dried yeast was suspended in 86.5 g of water to prepare a 13.5% (w/w) yeast slurry. The yeast was subjected to a boiling treatment for 15 minutes to be inactivated. The yeast was adjusted to a pH of 5.5 with 1N HCl or IN NaOH, and preincubated at 50°C; thereafter, 0.1 g of YL-T "Amano" L (Amano Enzyme Inc.) was added, and the yeast was treated at 60°C for 16 hours.

### (2) Nuclease Treatment

The lysed sample was adjusted to a pH of 5.0 with IN HCl or IN NaOH, and pre-incubated at 70°C; thereafter, 27 mg of Nuclease "Amano" G (Amano Enzyme Inc., nuclease activity: 7,000 U/g) was added, and the sample was treated at 70°C for 3 hours.

### (3) Deaminase Treatment

The nuclease-treated sample was adjusted to a pH of 5.5 with IN HCl or IN NaOH, and preincubated at 50°C; thereafter, 7 mg of Deamizyme G (Amano Enzyme Inc.; derived from the genus *Aspergillus*, deaminase activity: 50,000 U/mg) was added, and the sample was treated at 50°C for 3 hours. The deaminase-treated sample was heat-treated at 90°C for 15 minutes, and then centrifuged to collect a soluble fraction, which was analyzed for dry solids and the amount of nucleic acids.

### (4) Analysis of Dry Solids

The weight of dry solids obtained by treating 5 g of the soluble fraction at 105°C for 4 hours was measured, and the yield (%) of dry solids per gram of the dry yeast was calculated.

### (5) Analysis of Amount of Nucleic Acids

0.5 mL of purified water was added to 0.5 mL of the soluble fraction and subjected to microfiltration (MF); thereafter, the filtrate was analyzed for the amount of nucleic acids by HPLC analysis, and the IMP and GMP concentration (w/w%) per dry solids weight was calculated. The conditions for HPLC were as follows:
Column: Shim-Pack WAX-1 (Shimadzu Corporation)
Solvent: 50 mM KH₂PO₄ (pH 5.3)
Temperature: 45°C
Detection: 260 nm
Flow rate: 0.5 mL/min

### 2. Novel Method (Simultaneous Treatment with Nuclease and Deaminase)

### (1) Lysis Treatment

13.5 g of dried yeast was suspended in 86.5 g of water to prepare a 13.5% (w/w) yeast slurry. The yeast was subjected to a boiling treatment for 15 minutes to be inactivated. The yeast was adjusted to a pH of 5.5 with IN HCl or IN NaOH, and preincubated at 50°C; thereafter, 0.1 g of YL-T "Amano" L (Amano Enzyme Inc.) was added, and the yeast was treated at 60°C for 16 hours.

### (2) Simultaneous Treatment with Nuclease and Deaminase

The lysed sample was adjusted to a pH of 5.0 with IN HCl or IN NaOH, and pre-incubated at 60 or 65°C; thereafter, 27 mg of Nuclease E "Amano" 7 (Amano Enzyme Inc.; nuclease activity: 7,000 U/g) and 3.5 to 7 mg of Deamizyme T (Amano Enzyme Inc.; derived from *Streptomyces murinus*; deaminase activity: 50,000 U/mg) were added, and the sample was treated at 60 or 65°C for 6 hours. The treated sample was heat-treated at 90°C for 15 minutes, and then centrifuged to collect a soluble fraction, which was analyzed for dry solids and the amount of nucleic acids. The analysis conditions were the same as in the conventional method.

### <Results (Table 1)>

The yield of the yeast extract (dry solids) was compared between the conventional method and the novel method. Compared to the conventional method, the yield of the novel method increased by about 20% (when the treatment temperature during the simultaneous treatment was 60°C). The novel method provided a high yield even when the amount of the deaminase added was reduced. The analysis of the amount of nucleic acids also revealed that the yeast extract obtained by the novel method is rich in the umami components (GMP and IPM). That is, the novel method of treating with the nuclease and the deaminase simultaneously showed an unexpected effect in that not only the yield but also the quality of the yeast extract was improved.

**[Table 1]**

| | Amount of Deaminase Added | Treatment Temperature during Simultaneous Treatment | Dry Solids Yield (%) | IMP + GMP Concentration (w/w%) |
|---|---|---|---|---|
| Conventional Method (Separate Treatments) | 7mg | - | 25.2 | 3.25 |
| Simultaneous Treatment | 7mg | 60°C | 30.0 | 3.81 |
| | 4.9mg | | 30.4 | 3.72 |
| | 3.5mg | | 30.8 | 3.41 |
| | 7mg | 60°C | 26.5 | 3.80 |
| | 4.9mg | | 25.7 | 3.72 |
| | 3.5mg | | 25.8 | 3.63 |

### Industrial Applicability

The method of the present invention can simplify the process of producing a yeast extract, and can efficiently produce a yeast extract. The method can also be expected to improve the yield and quality (content of the umami components) of the yeast extract. The yeast extract obtained by the method of the present invention can be used to enhance or adjust the tastes of various foods and drinks.

The present invention can also be applied to the production of nucleic acid-based seasonings other than a yeast extract. Ingredients of the nucleic acid-based seasonings are not limited as long as they contain ribonucleic acids and/or ribonucleotides, and examples of usable ingredients include natural or processed products rich in ribonucleic acids, such as eggs (fish eggs and the like), milt of fish (salmon, blowfish, and the like), seafood, soybeans, and mushrooms (*shiitake* mushrooms, mushrooms, and the like).

This invention is in no way limited to the foregoing description of the embodiments of the invention and examples. This invention also includes various modifications that would be readily devised by those skilled in the art, without departing from the scope of the claims. All articles, unexamined patent application publications, and patent application publications expressly indicated herein are entirely incorporated by reference.

## Claims

1. A method for producing a yeast extract, comprising the step of treating a yeast cell lysate with a ribonuclease and AMP-deaminase simultaneously.

2. The method according to claim 1, wherein the ribonuclease is a ribonuclease derived from *Penicillium citrinum.*

3. The method according to claim 1 or 2, wherein the AMP-deaminase is AMP-deaminase derived from an actinomycete.

4. The method according to claim 3, wherein the actinomycete is an actinomycete of the genus *Streptomyces*

5. The method according to claim 4, wherein the actinomycete of the genus *Streptomyces* is *Streptomyces murinus, Streptomyces celluloflavus,* or *Streptomyces griseus.*

6. The method according to any one of claims 1 to 5, wherein treatment conditions are a temperature of 50 to 70°C and a pH of 4.5 to 8.0.

7. The method according to any one of claims 1 to 6, wherein the yeast cell lysate is a lysate of a yeast of the genus *Saccharomyces,* a yeast of the genus *Candida,* a yeast of the genus *Kluyveromyces,* a yeast of the genus *Pichia,* a yeast of the genus *Debaryomyces,* or a yeast of the genus *Zygosaccharomyces.*

8. A yeast extract obtained by the method according to any one of claims 1 to 7.

9. A food or drink comprising a yeast extract obtained by the method according to any one of claims 1 to 7.
